# EUROPEAN PATENT SPECIFICATION

(11) **EP 3 395 941 B1**
(45) Date of publication and mention of the grant of the patent: **10.03.2021**
(21) Application number: 16878818.0
(22) Date of filing: 21.12.2016
(51) Int. Cl.: C12N 5/00, C12N 5/071, C12N 5/074, C12N 5/0775, C12N 5/16, C12N 7/00, C12Q 1/02, C12Q 1/70, C12Q 1/18, G01N 33/50

(54) **VIRUS INFECTION MODEL, PREPARATION METHOD THEREFOR, AND UTILIZATION THEREOF**
VIRUSINFEKTIONSMODELL, HERSTELLUNGSVERFAHREN DAFÜR UND VERWENDUNG DAVON
MODÈLE D'INFECTION VIRALE, SON PROCÉDÉ DE PRÉPARATION, ET SON UTILISATION

(30) Priority: 22.12.2015 JP 2015249520
(43) Date of publication of application: 31.10.2018
(73) Proprietor: Public University Corporation Yokohama City University, Yokohama-shi, Kanagawa 236-0027 (JP)
(72) Inventor: TANIGUCHI, Hideki, Yokohama-shi Kanagawa 236-0004 (JP); MURATA, Soichiro, Yokohama-shi Kanagawa 236-0004 (JP); NIE, Yunzhong, Yokohama-shi Kanagawa 236-0004 (JP); MIYAKAWA, Kei, Yokohama-shi Kanagawa 236-0004 (JP); RYO, Akihide, Yokohama-shi Kanagawa 236-0004 (JP); TAKEBE, Takanori, Yokohama-shi Kanagawa 236-0004 (JP)
(74) Representative: J A Kemp LLP
(86) International application number: PCT/JP2016/088220
(87) International publication number: WO 2017/110931

(56) References cited:
- WO-A1-02/43477
- WO-A1-2013/047639
- JP-A- 2015 173 635
- JP-A- 2015 528 291
- PHILIP ROELANDT ET AL: "Human pluripotent stem cell-derived hepatocytes support complete replication of hepatitis C virus", JOURNAL OF HEPATOLOGY, ELSEVIER, AMSTERDAM, NL, vol. 57, no. 2, 29 March 2012 (2012-03-29) , pages 246-251, XP028426597, ISSN: 0168-8278, DOI: 10.1016/J.JHEP.2012.03.030 [retrieved on 2012-04-17]
- TAKESHI YOSHIDA ET AL: "Use of human hepatocyte-like cells derived from induced pluripotent stem cells as a model for hepatocytes in hepatitis C virus infection", BIOCHEMICAL AND BIOPHYSICAL RESEARCH COMMUNICATIONS, vol. 416, no. 1, 17 April 2012 (2012-04-17), pages 119-124, XP028392192, ISSN: 0006-291X, DOI: 10.1016/J.BBRC.2011.11.007 [retrieved on 2011-11-10]
- CHENG, LIN ET AL.: 'Induced Pluripotent Stem Cells(iPSCs) in the Modeling of Hepatitis C Virus Infection' CURRENT STEM CELL RESEARCH & THERAPY vol. 10, no. 3, 28 August 2015, pages 216 - 219, XP055507463
- TAKEBE, TAKANORI ET AL.: 'Vascularized and functional human liver from an iPSC-derived organ bud transplant' NATURE vol. 499, 03 July 2013, pages 481 - 485, XP055507466
- TREVISAN, MARTA ET AL.: 'Modeling Viral Infectious Diseases and Development of Antiviral Therapies Using Human Induced Pluripotent Stem Cell -Derived Systems' VIRUSES vol. 7, no. 7, July 2015, pages 3835 - 3856, XP055507471
- JIMENEZ , FRANCISCA M. ET AL.: 'Matrigel- embedded 3D culture of Huh-7 cells as a hepatocyte-like polarized system to study hepatitis C virus cycle' VIROLOGY vol. 425, 2012, pages 31 - 39, XP028414107

## Description

### TECHNICAL FIELD

The present invention relates to a virus infection model, a method for preparing the same, and use of the same.

### BACKGROUND ART

Recently, methods of generating human functional cells useful for drug discovery screening and regenerative medicine by directed differentiation using pluripotent stem cells, such as iPS cells capable of differentiating into various functional cells, have been attracting attention. To date, the research group of the present inventors has established a method of reconstructing human tissues/organs which have well-organized three-dimensional structures composed of vascular endothelial cells and mesenchymal cells as seen in adult tissues (Non-Patent Document No. 1: Takebe T. et al., Nature (2013) Vol. 499, pp 481-485; Patent Document No. 1: Method for Producing Tissue and Organ WO2013/047639).

On the other hand, infection models of hepatitis virus affecting humans have been conventionally prepared using primary cultures of human hepatocytes or various tumor cells (Non-Patent Document No. 2: Gripon P et al. Virol 1995; 213:292-299; Non-Patent Document No. 3: Watashi K et al., JBC. 2013 288: 31725-31727; Non-Patent Document No. 4: Yang D et al. PNAS 2014 E1264-E1273). However, the only system reported to date in which an *in vitro* reconstituted three-dimensional tissue is to be infected with hepatitis virus is one that uses tumor cells (Non-Patent Document No. 5: Molina-Jimenez F et al., Virology 2012 425; 31-9).

Models in which primary cultures of human hepatocytes are infected with hepatitis virus (HBV: hepatitis B virus) have the following advantages and disadvantages.
- Susceptibility to HBV infection without over-expression of NTCP known as an HBV receptor, and so forth;
- Limited supply of hepatocytes; Non-proliferation of the hepatocytes;
- Low reproducibility (big difference between lots).

Further, models in which hepatoma cell lines have been infected with hepatitis virus have the following advantages and disadvantages.
- The cells are capable of proliferation without lot-to-lot differences.
- A large quantity of virus (2,000-6,000 Geq/cell) is needed for establishment of infection.
- Hepatocyte-specific markers are not expressed because the cells are tumor cells.

One problem with virus infection tests is that recapitulation of viral life cycle is difficult in the current culture system. Conventional primary cultures of hepatocytes are not susceptible to re-infection. As an infection model susceptible to re-infection, only human hepatocytes isolated from chimeric mice with a humanized liver have been reported (Non-Patent Document No. 6: Ishida Y, Am J Patho, Vol. 185, No.5, May 2015, pp.1275-1285). Other infection models are prepared by infecting tumor cells with virus (Non-Patent Document No. 7: Yang D, PNAS E1264-E1273 published online March 10, 2014; Non-Patent Document No. 8: Sai LT, JMII (2014)xx, 1-6).

Japanese patent application publication no. JP 2015-173635 (Patent Document No. 2) discloses a hepatitis tissue body which imitates a hepatic tissue infected with a hepatitis virus, contains an endothelial cell having a tubular structure, and a cell mass formed on the endothelial cell having the tubular structure, in which at least a part of a cell constituting the cell mass holds a hepatitis virus.

### PRIOR ART LITERATURE

### Non-Patent Documents

Non-Patent Document No. 1: Nature (2013) VOL.499, pp 481-485
Non-Patent Document No. 2: Gripon P et al. Virol 1995; 213:292-299
Non-Patent Document No. 3: Watashi K et al., JBC. 2013 288:31725-31727
Non-Patent Document No. 4: Yang D et al. PNAS 2014 E1264-E1273
Non-Patent Document No. 5: Molina-Jimenez F et al. Virology 2012 425; 31-9
Non-Patent Document No. 6: Ishida Y, Am J Patho, Vol. 185, No.5, May 2015, pp.1275-1285
Non-Patent Document No. 7: Yang D, PNAS E1264-E1273 published online March 10, 2014
Non-Patent Document No. 8: Sai LT, JMII (2014)xx, 1-6

### Patent Document

Patent Document No. 1: WO2013/047639
Patent Document No. 2: JP 2015-173635

### DISCLOSURE OF THE INVENTION

### PROBLEM FOR SOLUTION BY THE INVENTION

Conventional hepatitis virus infection models have the following drawbacks.
- The viral life cycle seen in hepatitis virus infection to humans, especially multi-round infection kinetics, could not be recapitulated.
- High concentrations of virus could not be released from the infected cells to the culture supernatant.
- Use of tumor cells made it impossible to examine functional disorders of the liver.

The present invention aims at solving these problems.

### MEANS TO SOLVE THE PROBLEM

As a result of intensive and extensive efforts toward the solution of the above problems, the present inventors have found a technique for preparing a completely novel virus infection model using a human tissue/organ generated from pluripotent stem cells, etc. Briefly, the present inventors combined pluripotent stem cells (e.g., human iPS cells) with mesenchymal cells and vascular endothelial cells, to thereby reconstitute three-dimensional human liver buds; as a result, the multiplicity of infection of various organotropic viruses was outstandingly increased, leading to a successful construction of an infection model closely simulating an infection to the human liver. The present invention is expected to help advance the screenings in drug discovery and the elucidation of viral infection mechanism, eventually leading to the development of new drugs against various hepatitis virus infections and the elucidation of new mechanisms behind their pathogenesis.

The gist of the present invention is as described below.
(1) A method for constructing a hepatitis virus infection model capable of recapitulating a viral life cycle, by infecting a liver bud with hepatitis virus, wherein said liver bud is formed by co-culturing liver cells derived from human iPSC with human vascular endothelial cells and human mesenchymal cells.
(2) The method of (1) above, wherein the vascular endothelial cells are human umbilical vein endothelial cells.
(3) The method of (1) above, wherein the mesenchymal cells are human bone marrow-derived mesenchymal cell.
(4) The method of (1) above, wherein the infecting hepatitis virus is hepatitis B virus, hepatitis C virus or hepatitis E virus.
(5) A hepatitis virus infection model capable of recapitulating a viral life cycle, comprising a hepatitis virus-infected liver bud, wherein the liver bud is formed by co-culturing liver cells derived from human iPSC with human vascular endothelial cells and human mesenchymal cells.
(6) The virus infection model of (5) above, wherein the infecting hepatitis virus is hepatitis B virus, hepatitis C virus or hepatitis E virus.
(7) A method of screening for substances with antiviral activity, comprising using the virus infection model of (5) above.
(8) A method of screening for substances with antiviral activity, comprising using the virus infection model of (6) above, wherein the infecting virus is hepatitis B virus, hepatitis C virus or hepatitis E virus.

To date, the research group of the present inventors has succeeded in constructing a platform technology for preparing a human tissue/organ in which a vascular system is appropriately located in a way that has never been achieved by conventional techniques (WO2013/047639 Method for Producing Tissue and Organ).

In the present invention, a human liver bud prepared by the above-described platform technology is infected with hepatitis virus and this enables the following to be achieved.
- To recapitulate the viral life cycle seen in hepatitis virus infection to humans, especially multi-round infection kinetics.
- To release high concentrations of virus from infected cells into the culture supernatant
- To examine functional disorders of the liver.

It is anticipated that the present invention can be applied to *in vitro* screenings in drug discovery in a more convenient and efficient manner than conventionally performed.

### EFFECT OF THE INVENTION

Since liver buds can be produced uniformly and in large quantities, the present invention can eliminate inter-lot variability as seen in conventional primary cultures of hepatocytes, making it possible to produce a large quantity of the virus-infected human liver tissue necessary for drug discovery and development.

The present specification refers to the contents disclosed in the specification and/or the drawings of Japanese Patent Application No. 2015-249520 based on which the present patent application claims priority.

### BRIEF DESCRIPTION OF THE DRAWINGS

[Fig. 1] Upper row: photographs of a human liver bud prepared by three-dimensionally culturing human iPS cells, vascular endothelial cells and undifferentiated mesenchymal cells, as observed with an inverted optical microscope at days 1, 7 and 15 after preparation.
   Bottom row: photographs of the human liver bud infected with HBV, as observed with an inverted optical microscope at days 10, 20 and 30 post infection.
[Fig. 2] A graph comparing HBV DNA copy numbers in culture supernatants between two cases where human iPS cell-derived hepatocytes and human liver buds were infected with HBV.
[Fig. 3] A graph comparing the intracellular HBV 3.5k RNA quantities between two cases where human iPS cell-derived hepatocytes and human liver buds were infected with HBV
[Fig. 4] This figure shows HBV infection inside the human liver bud. The liver bud at days 10, 20 and 30 post infection is immunologically stained with HBc antibody.
[Fig. 5] A graph comparing HBV DNA copy numbers in culture supernatants of primary human hepatocytes (PXB cells) between two cases where PXB cells were infected with the culture supernatant of human liver buds at day 20 post infection and with a non-infected group.
[Fig. 6] A graph comparing intracellular HBV covalently closed circular DNA copy numbers between two cases where human iPS cell-derived hepatocytes and human liver buds were infected with HBV Compared to human iPS cell-derived hepatocytes, human liver buds had a noticeably greater number of HBV covalently closed circular DNA copies.
[Fig. 7] Electron micrographs comparing human liver bud at day 10 post infection with non-infected human liver bud. The HBV-infected human liver bud was observed to have fewer hepatocyte microvilli (indicated by arrow heads).
[Fig. 8] This figure shows that infection of human liver bud with HBV is inhibited by various drugs. Con: control group; IFN-α: interferon α 1000U/ml; IFN-γ: interferon γ 1000U/ml; PreS1: HBV surface antigen PreS1 peptide 100 nM; ETV: entecavir 1.8 µM. *p<0.05, **p<0.01, ***p<0.0001 vs Con.

### BEST MODES FOR CARRYING OUT THE INVENTION

Hereinbelow, the present invention will be described in detail.

The present invention provides a method for constructing a hepatitis virus infection model capable of recapitulating a viral life cycle, by infecting a liver bud with hepatitis virus, wherein said liver bud is formed by co-culturing liver cells derived from human iPSC with human vascular endothelial cells and human mesenchymal cells.

As disclosed herein, the term "cell condensate" refers to one that is formed by culturing a tissue or organ cell *in vitro.* Such a cell condensate is disclosed in, for example, WO2015/129822 (Method for generating cell condensate for self-organization). In the cell condensate, cell-cell interactions take place in such a close manner that a biological environment as occurs in the womb is recapitulated. As a consequence, induction of early differentiation into organ progenitor cells occurs efficiently and this would improve the frequency and number of organ progenitor cells present. The cell condensate may be one in which cells adhere to each other so strongly that it can be collected in a non-destructive manner.

The cell condensate described above is of a concept that generally encompasses organ buds and organoids [organ bud (WO2013/047639), liver bud, liver diverticula, liver organoid, pancreatic (dorsal or ventral) buds, pancreatic diverticula, pancreatic organoid, intestinal bud, intestinal diverticula, intestinal organoid (K. Matsumoto et al. Science. 19; 294 (5542): 559-63 (2001)]. While the cell condensate is independent of the types of constituent cells and the number of such types, organ buds correspond to cell condensates that are formed at an early stage of organogenesis and are in principle composed of the following three types of cells: functional cells that constitute organs or tissues (or undifferentiated cells which will differentiate into functional cells); vascular cells; and mesenchymal cells. Organoids are solely composed of cells that constitute epithelial tissues and they are basically of a small size (1 mm or less).

The cell condensate undergoes self-organization to form a three-dimensional tissue structure provided with higher structures, whereby progenitor cells can be directed to terminal differentiation. Self-organization may be performed either *in vivo* or *in vitro.* For example, when the cell condensate is transplanted into a living body, vascular networks are formed, blood perfusion is induced, and self-organization into a higher tissue with a complex structure occurs, enabling the preparation of tissues/organs that have a highly ordered tissue structure comparable to that of adult tissues. With such a cell condensate, it may be possible to prepare a higher tissue that is provided not only with a vascular network but also with additional higher structures such as ureteral structure, biliary structure, tracheal structure, etc. Further, there are many organs including the liver which, in order to exhibit their functions, essentially require the reconstitution of not only themselves but also associations with other organs; in the case of the liver, not only itself but also the junctions with the bile and pancreatic ducts as well as the connection to the duodenum must be reconstituted in order that it exhibits its function.

The cell condensate may be formed by culturing a tissue or organ cell with vascular endothelial cells or mesenchymal cells *in vitro.* A method for preparing such a cell condensate is disclosed in WO2015/129822 (Method for generating cell condensate for self-organization), but other preparation methods may also be used.

The cell condensate formed by culturing a tissue or organ cell *in vitro* may be an organ bud.

As disclosed herein, the term "organ bud" means a structure capable of differentiating into an organ through maturing, the structure comprising tissue or organ cells, vascular endothelial cells and mesenchymal cells (undifferentiated mesenchymal cells or cells differentiated therefrom). Whether a structure is an organ bud or not can be determined, for example, by transplanting the structure into an organism and examining whether or not it is capable of differentiating into an organ of interest (the structure can be judged as organ bud if it has differentiated into the organ of interest); and/or by examining whether or not the structure comprises all of the above-described three types of cells, i.e., tissue or organ cells, vascular endothelial cells and mesenchymal cells (the structure can be judged as organ bud if it comprises all of the three types of cells). The organ bud may be one which differentiates into an organ such as kidney, heart, lung, spleen, esophagus, stomach, thyroid, parathyroid, thymus, gonad, brain, spinal cord or the like. Preferably, the organ bud is one which differentiates into an endodermal organ such as one which differentiates into liver (liver bud), one which differentiates into pancreas (pancreas bud), or one which differentiates into intestinal tract. Whether a certain structure is an organ bud which differentiates into an endodermal organ or not can be determined by examining the expression of marker proteins

(if any one or more of the marker proteins described later are expressed, the organ bud can be judged as the organ bud of interest). For example, HHEX, SOX2, HNF4A, AFP, ALB and the like are markers for liver bud; PDX1, SOX17, SOX9 and the like are markers for pancreas bud; and CDX2, SOX9 and the like are markers for organ buds which differentiate into intestinal tract. Among the terms used by those skilled in the art, the following are included in the organ bud of the disclosure: liver bud, liver diverticula, liver organoid, pancreatic (dorsal or ventral) buds, pancreatic diverticula, pancreatic organoid, intestinal bud, intestinal diverticula, intestinal organoid (K. Matsumoto, et al. Science. 19; 294 (5542): 559-63 (2001)) and so on.

An organ bud may be formed by culturing tissue or organ cells with vascular endothelial cells and mesenchymal cells *in vitro.* A method for preparing an organ bud is disclosed in WO2013/047639 (Method for Producing Tissue and Organ), but other preparation methods may also be used.

As used herein, the term "tissue or organ cell" means functional cells constituting tissues or organs, or undifferentiated cells which differentiate into functional cells. Examples of "undifferentiated tissue or organ cell" include, but are not limited to, cells capable of differentiating into an organ such as kidney, heart, lung, spleen, esophagus, stomach, thyroid, parathyroid, thymus, gonad, brain or spinal cord; cells capable of differentiating into an ectodermal organ such as brain, spinal cord, adrenal medulla, epidermis, hair/nail/dermal gland, sensory organ, peripheral nerve or lens; cells capable of differentiating into a mesodermal organ such as kidney, urinary duct, heart, blood, gonad, adrenal cortex, muscle, skeleton, dermis, connective tissue or mesothelium; and cells capable of differentiating into an endodermal organ such as liver, pancreas, intestinal tract, lung, thyroid, parathyroid or urinary tract. Whether or not a cell is capable of differentiating into an ectodermal organ, mesodermal organ or endodermal organ can be determined by examining the expression of marker proteins (if any one or more of marker proteins are expressed, the cell can be judged as a cell capable of differentiating into an endodermal organ). For example, in cells capable of differentiating into liver, HHEX, SOX2, HNF4A, AFP, ALB and the like are markers; in cells capable of differentiating into pancreas, PDX1, SOX17, SOX9 and the like are markers; in cells capable of differentiating into intestinal tract, CDX2, SOX9 and the like are markers; in cells capable of differentiating into kidney, SIX2 and SALL1 are markers; in cells capable of differentiating into heart, NKX2-5, MYH6, ACTN2, MYL7 and HPPA are markers; in cells capable of differentiating into blood, C-KIT, SCA1, TER119 and HOXB4 are markers; and in cells capable of differentiating into brain or spinal cord, HNK1, AP2, NESTIN and the like are markers. Among the terms used by those skilled in the art, the following are included in the "undifferentiated tissue or organ cell" of the disclosure: hepatoblast, hepatic progenitor cells, hepatic precursor cells, pancreatoblast, pancreatic progenitors, pancreatic progenitor cells, pancreatic precursor cells, endocrine precursors, intestinal progenitor cells, intestinal precursor cells, intermediate mesoderm, metanephric mesenchymal precursor cells, multipotent nephron progenitor, renal progenitor cells, cardiac mesoderm, cardiovascular progenitor cells, cardiac progenitor cells (JR. Spence, et al. Nature.; 470(7332):105-9.(2011); Self, et al. EMBO J.; 25(21): 5214-5228.(2006); J. Zhang, et al. Circulation Research.; 104: e30-e41(2009); G. Lee, et al. Nature Biotechnology 25, 1468-1475 (2007)) and so on. Undifferentiated tissue or organ cells may be collected from tissues or organs, or may be prepared from pluripotent stem cells such as induced pluripotent stem cells (iPS cells) or embryonic stem cells (ES cells) according to known methods. Moreover, undifferentiated tissue or organ cells may be such cells as primitive gut endoderm cells (PGECs) (Japanese Patent No. 5777127) which are at an intermediate stage of differentiation from pluripotent stem cells (e.g., iPS cells) into tissues or organs. PGECs are capable of differentiating into hepatocytes, pancreatic cells and enterocytes (have high differentiation function), do not express markers associated with the malignancy of cancer (are highly safe), and may be prepared from iPS cells by directed differentiation without using feeder cells. Therefore, PGECs have the advantage of even allowing for clinical application. Furthermore, mass preparation of PGECs is possible. PGECs may be prepared according to the method disclosed in Japanese Patent No. 5777127. Alternatively, endodermal cells induced from pluripotent stem cells (e.g., iPS cells) cultured in an activin-, Wnt 3a- and NaB-supplemented serum-free medium may be used. To give further examples, organ cells capable of differentiating into liver may be prepared as previously described (K. Si-Taiyeb, et al. Hepatology, 51 (1): 297- 305(2010); T. Touboul, et al. Hepatology. 51 (5): 1754-65 (2010)); organ cells capable of differentiating into pancreas may be prepared as previously described (D. Zhang, et al. Cell Res.;19(4):429-38 (2009)); organ cells capable of differentiating into intestinal tract may be prepared as previously described (J. Cai, et al. J Mol Cell Biol.; 2(1):50-60 (2010); R. Spence, et al. Nature.; 470 (7332): 105-9 (2011)); cells capable of differentiating into heart may be prepared as previously described (J. Zhang, et al. Circulation Research.; 104: e30-e41(2009); and cells capable of differentiating into brain or spinal cord may be prepared as previously described (G. Lee, et al. Nature Biotechnology 25, 1468 - 1475 (2007)). Examples of "differentiated tissue or organ cell" include, but are not limited to, endocrine cells of pancreas, pancreatic duct epithelial cells of pancreas, hepatocytes of liver, epithelial cells of intestinal tract, tubular epithelial cells of kidney, podocytes of kidney, cardiomyocytes of heart, lymphocytes and granulocytes of blood, erythrocytes, neurons and glial cells of brain, and neurons and Schwann cells of spinal cord. As tissue or organ cells, human-derived cells are mainly used. However, tissue or organ cells derived from non-human animals (e.g., animals used, for example, as experimental animals, pet animals, working animals, race horses or fighting dogs; more specifically, mouse, rat, rabbit, pig, dog, monkey, cattle, horse, sheep, chicken, shark, devilfish, ratfish, salmon, shrimp, crab or the like) may also be used.

In the present invention, the term "vascular endothelial cell" means cells constituting vascular endothelium or cells capable of differentiating into such cells. Whether a cell is vascular endothelial cell or not can be determined by examining the expression of marker proteins such as TIE2, VEGFR-1, VEGFR-2, VEGFR-3 and CD41 (if any one or more of the above-listed marker proteins are expressed, the cell can be judged as vascular endothelial cell). The vascular endothelial cell used in the present invention may be either differentiated or undifferentiated. Whether a vascular endothelial cell is a differentiated cell or not can be determined by means of CD31 and CD144. Among the terms used by those skilled in the art, the following are included in the "vascular endothelial cell" used in the present invention endothelial cells, umbilical vein endothelial cells, endothelial progenitor cells, endothelial precursor cells, vasculogenic progenitors, hemangioblast (HJ. Joo, et al. Blood. 25;118(8):2094-104 (2011)) and so on. Preferable vascular endothelial cells are those derived from umbilical vein. As vascular endothelial cells, human-derived cells are used.

In the present invention, the term "mesenchymal cell" means connective tissue cells that are mainly located in mesoderm-derived connective tissues and which form support structures for cells that function in tissues. The "mesenchymal cell" is a concept that encompasses those cells which are destined to, but are yet to, differentiate into mesenchymal cells. Mesenchymal cells used in the present invention may be either differentiated or undifferentiated. Whether a certain cell is an undifferentiated mesenchymal cell or not may be determined by examining the expression of marker proteins such as Stro-1, CD29, CD44, CD73, CD90, CD105, CD133, CD271 or Nestin (if any one or more of the above-listed marker proteins are expressed, the cell can be judged as an undifferentiated mesenchymal cell). A mesenchymal cell in which none of the above-listed markers are expressed can be judged as a differentiated mesenchymal cell. Among the terms used by those skilled in the art, the following are included in the "mesenchymal cell" used in the present invention mesenchymal stem cells, mesenchymal progenitor cells, mesenchymal cells (R. Peters, et al. PLoS One. 30; 5(12):e15689 (2010)) and so on. Preferable mesenchymal cells are mesenchymal cells derived from bone marrow (especially, mesenchymal stem cells). As mesenchymal cells, human-derived cells are used.

Culture ratios of three cell types in coculture are not particularly limited as long as they are within the range that enables the formation of liver buds. Preferable cell count ratio is 10 : 10-5 : 0.1-3 for liver cell : human vascular endothelial cell : human mesenchymal cell. Liver buds of approximately 50 µm to 3 mm in size may be formed by coculturing approx. 400,000 liver cells, approx. 200,000 to 400,000 human vascular endothelial cells and approx. 20,000 to 120,000 human mesenchymal cells.

The medium used for culture may be any medium that enables the formation of cell condensates (preferably, organ buds) and examples that are preferably used include a medium for culturing vascular endothelial cells, a medium for culturing tissue or organ cells, and a mixture of these two media. As a medium for culturing vascular endothelial cells, any medium may be used but, preferably, a medium containing at least one of the following substances may be used: hEGF (recombinant human epidermal growth factor), VEGF (vascular endothelial growth factor), hydrocortisone, bFGF, ascorbic acid, IGF1, FBS, antibiotics (e.g., gentamycin or amphotericin B), heparin, L-glutamine, phenol red and BBE. Specific examples of media that may be used for culturing vascular endothelial cells include, but are not limited to, EGM-2 BulletKit (Lonza), EGM BulletKit (Lonza), VascuLife EnGS Comp Kit (LCT), Human Endothelial-SFM Basal Growth Medium (Invitrogen) and human microvascular endothelial cell growth medium (TOYOBO). As a medium for culturing tissue or organ cells, any medium may be used but in the case where the organ cell is a hepatocyte, a medium containing at least one of ascorbic acid, BSA-FAF, insulin, hydrocortisone and GA-1000 may preferably be used. As a medium for culturing hepatocytes, HCM BulletKit (Lonza) from which hEGF (recombinant human epidermal growth factor) has been removed or RPMI1640 (Sigma-Aldrich) to which 1% B27 Supplements (GIBCO) and 10 ng/mL hHGF (Sigma-Aldrich) have been added may typically be used. As regards the formation of human liver buds, a 1:1 mixture of GM BulletKit (Lonza) and HCM BulletKit (Lonza) from each of which hEGF has been removed and which are each supplemented with dexamethasone, oncostatin M and HGF has been found effective for maturation of liver buds.

Although scaffold materials need not be used for culturing cells, a cell mixture may advantageously be cultured on a gel-like support that allows mesenchymal cells to contract.

Contraction of mesenchymal cells may be confirmed, for example, by noting the formation of a 3D tissue morphologically (either under microscope or with the naked eye) or by showing that the tissue is strong enough to retain its shape as it is collected with a spatula or the like (Takebe et al. Nature 499 (7459), 481-484, 2013).

The support may advantageously be a gel-like substrate having an appropriate stiffness [e.g., a Young's modulus of 200 kPa or less (in the case of a Matrigel-coated gel of a flat shape); however, the appropriate stiffness of the support may vary depending on the coating and shape]. Examples of such substrates include, but are not limited to, hydrogels (such as acrylamide gel, gelatin and Matrigel). The stiffness of the support need not be uniform and may vary with the shape, size and quantity of a cell condensate of interest so that it can be provided with a spatial/temporal gradient or can be patterned. In the case where the stiffness of the support is uniform, it is preferably 100 kPa or less, more preferably 1-50 kPa. The gel-like support may be planar, or alternatively, the side on which culture is to be performed may have a U- or V-shaped cross section. If the side of the gel-like support on which culture is to be performed has a U- or V-shaped cross section, cells tend to gather on the culture surface and a cell condensate can advantageously be formed from a smaller number of cells and/or tissues. Moreover, the support may be modified chemically or physically. Examples of modifying substances include, but are not limited to, Matrigel, laminin, entactin, collagen, fibronectin and vitronectin.

One example of the gel-like culture support that is provided with a spatial gradient of stiffness is a gel-like culture support that is stiffer in the central part than in the peripheral part. The stiffness of the central part is appropriately 200 kPa or less and it suffices that the peripheral part is softer than the central part. Appropriate values for the stiffness of the central and peripheral parts of the substrate are variable with the coating and the shape. Another example of the gel-like culture support that is provided with a spatial gradient of stiffness is a gel-like culture support that is stiffer in the peripheral part than in the central part.

One example of the patterned, gel-like culture support is a gel-like culture support having one or more patterns in which the central part is stiffer than the peripheral part. The stiffness of the central part is appropriately 200 kPa or less and it suffices that the peripheral part is softer than the central part. Appropriate values for the stiffness of the central and peripheral parts of the substrate are variable with the coating and the shape. Another example of the patterned, gel-like culture support is a gel-like culture support having one or more patterns in which the peripheral part is stiffer of than the central part. The stiffness of the peripheral part is appropriately 200 kPa or less and it suffices that the central part is softer than the peripheral part. Appropriate values for the stiffness of the central and peripheral parts of the substrate are variable with the coating and the shape.

The temperature during culture is not particularly limited but it is preferably 30-40°C and more preferably 37°C.

The culture period is not particularly limited but it is preferably 3-10 days and more preferably 6 days.

A hepatitis virus infection model may be prepared by infecting a liver bud formed by the above-described method with a hepatitis virus. This model is capable of recapitulating a viral life cycle, especially multi-round infection kinetics. Moreover, this model achieves high concentrations of hepatitis virus release from infected cells to the culture supernatant. As will be shown in the Example described later, the level of intracellular HBV RNA in the virus infection model of the present invention (HBV-infected liver bud) increased up to day 30 post infection, and HBV DNA was observed in the culture supernatant. Further, the virus released into the culture supernatant of the virus infection model of the present invention was capable of infecting other cells. In other words, the life cycle of the virus could be recapitulated. Further, a markedly high HBV closed circular DNA was noted in the virus infection model of the invention (HBV-infected liver bud). Still further, hepatocyte microvilli were reduced in the virus infection model of the present invention (HBV-infected liver bud).

The types of hepatitis virus which may be used in the present invention include, but are not limited to, hepatitis B virus, hepatitis C virus and hepatitis E virus.

The liver bud to be infected with a hepatitis virus may advantageously be one prepared by mixing, for example, liver cells derived from human iPSC, human vascular endothelial cells and human mesenchymal cells, coculturing the resultant mixture in a medium containing DMSO for 7 days and further coculturing in a medium containing HGF, OSM and DEX for 7 days. For infection with HBV, the liver bud may, for example, be infected with about 500 copies of HBV per liver cell, and then cultured for about 30 days. A medium containing PEG8000 may be used for the culture.

The present invention also provides a hepatitis virus infection model capable of recapitulating a viral life cycle, comprising a hepatitis virus-infected liver bud, wherein the liver bud is formed by co-culturing liver cells derived from human iPSC with human vascular endothelial cells and human mesenchymal cells.

The liver bud, the virus and the virus-infected liver bud are as described above. The types of hepatitis virus which may be used in the present invention include, but are not limited to, hepatitis B virus, hepatitis C virus and hepatitis E virus.

The present invention also provides a method of screening for substances with antiviral activity, comprising using the above-described hepatitis virus infection model. The types of hepatitis virus which may be used in the present invention include, but are not limited to, hepatitis B virus, hepatitis C virus and hepatitis E virus.

One example of the method of screening for substances with antiviral activity will be described below. To begin with, the above-described liver bud is infected with a hepatitis virus, and then a test substance is supplied thereto. Alternatively, a test substance is mixed into a liver bud under preparation. Quantities of hepatitis virus in the liver bud culture supernatant or in the liver bud *per se* are compared between two groups, one supplied with the test substance and the other not supplied with the test substance. Alternatively, test substances which reduce the quantity of hepatitis virus in the culture supernatant or in the liver bud by 50% or more are identified. Test substances are not particularly limited and may include, but are not limited to, low molecular weight compounds, peptides, nucleic acids, extracts from natural products, and inorganic compounds. The quantities of hepatitis virus in liver bud culture supernatant or intracellular hepatitis virus from day 1 to day 30 after the supply of test substance are compared to the corresponding quantities in the control group which did not receive the test substance. It is desirable to select those test substances which reduce the quantity of hepatitis virus to 30% or less of the quantity in the control group not supplied with the test substance. This method enables the screening for hepatitis viral therapeutics or prophylactics.

Target substances for screening may be proteins, polypeptides, oligopeptides, nucleic acids (including natural and artificial nucleic acids), low molecular weight organic compounds, inorganic compounds, cell extracts, and extracts from animals or plants, soils and so forth. The substance may be either a natural product or a synthesized product.

### EXAMPLES

Hereinbelow, the present invention will be described in more detail with reference to the following Example.

### [Example 1]

### Experimental Methods

Human liver buds were prepared according to the "Method for Preparing Tissue and Organ" (WO2013/047639).
(1) Preparation of human hepatic endoderm cells
   Human iPS cells (for example, human skin-derived TkDA3 hiPSC clone (provided by the University of Tokyo)) were cultured in activin-, Wnt 3a- and NaB-supplemented serum-free medium to thereby obtain endodermal cells (definitive endoderms: DEs).
(2) Preparation of human liver buds
   The endodermal cells obtained, vascular endothelial cells (human umbilical vein-derived endothelial cells) (Lonza, Basel, Switzerland) and undifferentiated mesenchymal cells (human bone marrow-derived mesenchymal stem cells) (Lonza, Basel, Switzerland) were mixed at 10:7:1 (400 cells: 280 cells: 40 cells per organ bud) to prepare human liver buds (120-150 µm in size).
(3) Infection of human liver buds with HBV
   The human liver buds prepared in (2) above were cultured in DMSO-containing medium for 7 days and further cultured in HGF-, OSM- and DEX-containing medium for 7 days, followed by infection with HBV. HBV was recovered from culture supernatant of HepG2.2.15 cells which had been prepared by transferring the intact HBV genome (genotype D) into human hepatoma cell line HepG2 by lipofection. The liver buds were infected with approx. 500 copies of HBV per endodermal cell in 4% PEG8000-containing medium and cultured for about 30 days.
(4) Preparation of human iPS cell-derived hepatocytes and HBV infection
   The endodermal cells obtained in (1) above were cultured in KO-DMEM-, KSR- and DMSO-containing medium for 7 days to thereby obtain immature hepatocytes (IHs). The resultant cells were then cultured in HGF-, OSM- and DEX-containing medium for another 7 days to thereby obtain human iPS cell-derived hepatocytes (mature hepatocytes: MHs). The human iPS cell-derived hepatocytes were infected with the same HBV as used in (3) above. Approx. 500 copies of HBV per cell and 4% PEG8000-containing medium were used for infection.
(5) Culture supernatants of human liver buds and human iPS-cell derived hepatocytes were recovered at days 10, 20 and 30 post HBV infection, followed by measurement of HBV DNA.
(6) Human liver buds and human iPS-cell derived hepatocytes were recovered at days 10, 20 and 30 post HBV infection, followed by measurement of HBV 3.5k RNA.
(7) The inside of human liver buds was immunostained with HBc antibody at days 10, 20 and 30 post HBV infection.
(8) Human primary hepatocytes (PXB cells; Phoenix Bio) were infected with the culture supernatant of human liver buds at day 20 post HBV infection.
(9) Human liver buds and human iPS-cell derived hepatocytes were recovered at day 20 post HBV infection, followed by measurement of covalently closed circular DNA (cccDNA).
(10) Human liver buds at day 10 post HBV infection and non-HBV-infected human liver buds in the corresponding period were fixed with paraformaldehyde and glutaraldehyde, and electron micrographs were taken.
(11) Human liver buds were infected with HBV (5,000 copies per cell). Interferon α (1000 U/ml), interferon γ (1000 U/ml), PreS1 peptide (100 nM), and entecavir (1.8 µM) were added to the medium, in which the liver buds were cultured for 10 days. Intracellular HBV 3.5k RNA and HBV DNA in culture supernatant were measured.

### Results

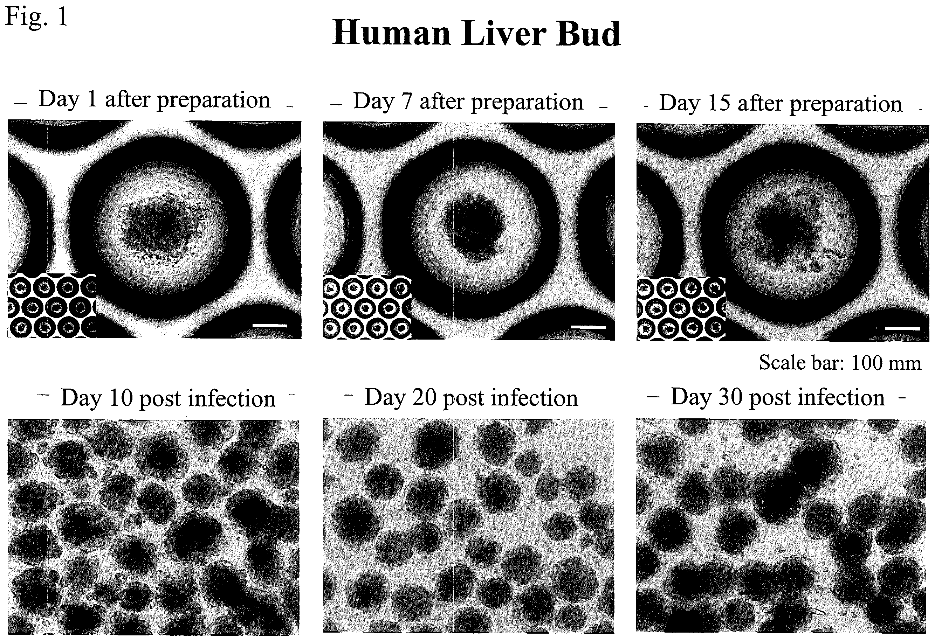

The results are shown in Figs. 1 to 8.

Fig. 1, upper row shows photographs of a human liver bud prepared by three-dimensionally culturing human iPS cells, vascular endothelial cells and undifferentiated mesenchymal cells, which were taken with an inverted microscope at days 1, 7 and 15 after preparation. Even after 15 days from the preparation, the liver bud retains a spherical 3D structure. Fig. 1, bottom row shows photographs of the human liver bud infected with HBV, which were taken with an inverted microscope at days 10, 20 and 30 post infection. The morphology of the liver bud is retained up to 30 days post infection.

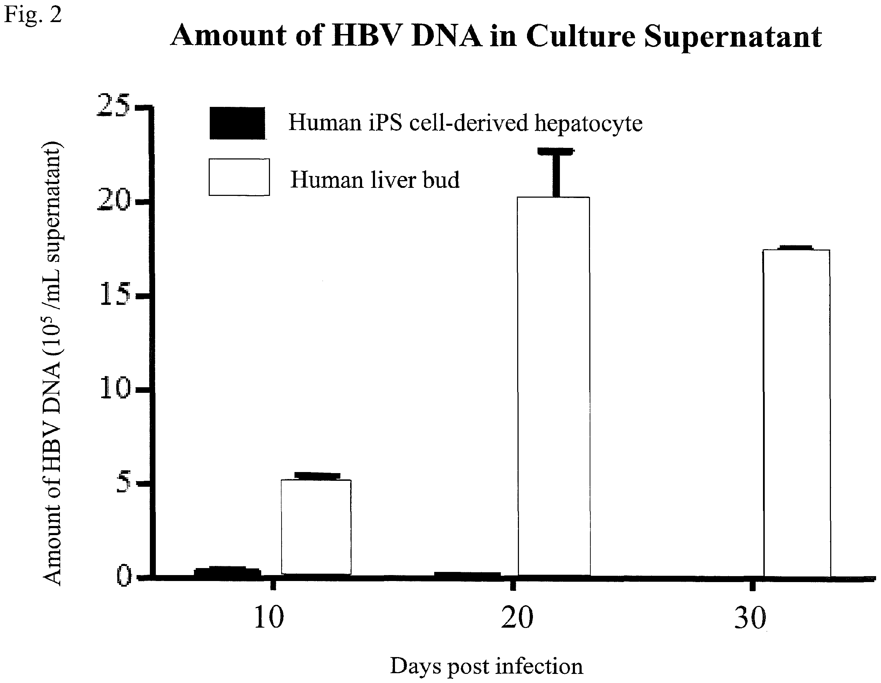

Fig. 2 is a graph comparing HBV DNA copy numbers in culture supernatants between two cases where human iPS cell-derived hepatocytes and human liver buds were infected with HBV Markedly high HBV release was noted in human liver buds, compared with human iPS-derived hepatocytes.

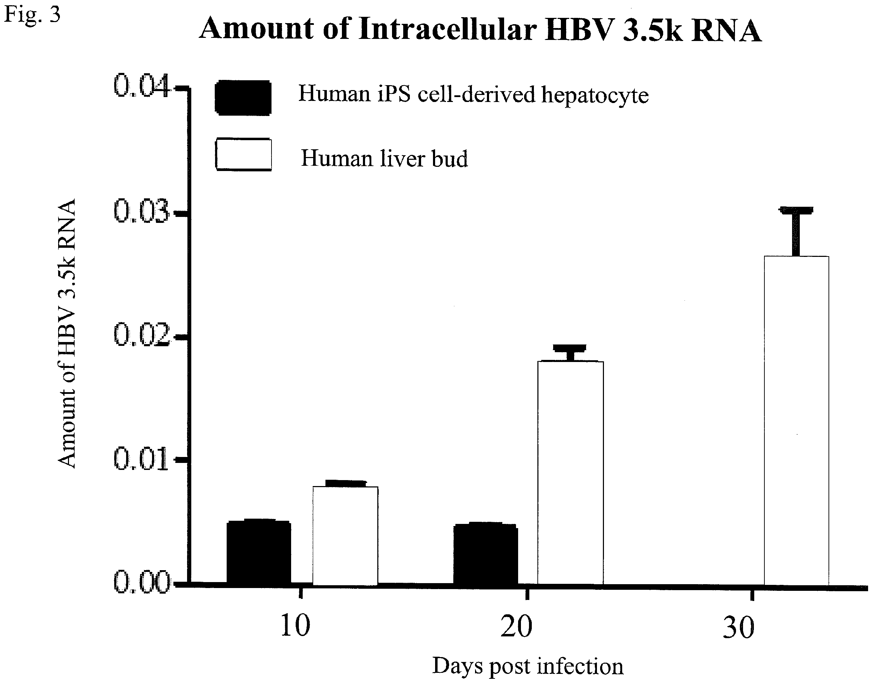

Fig. 3 is a graph comparing the intracellular HBV 3.5k RNA quantities between two cases where human iPS cell-derived hepatocytes and human liver buds were infected with HBV. Markedly high HBV 3.5k RNA was noted in cells of human liver buds, compared with human iPS-derived hepatocytes.

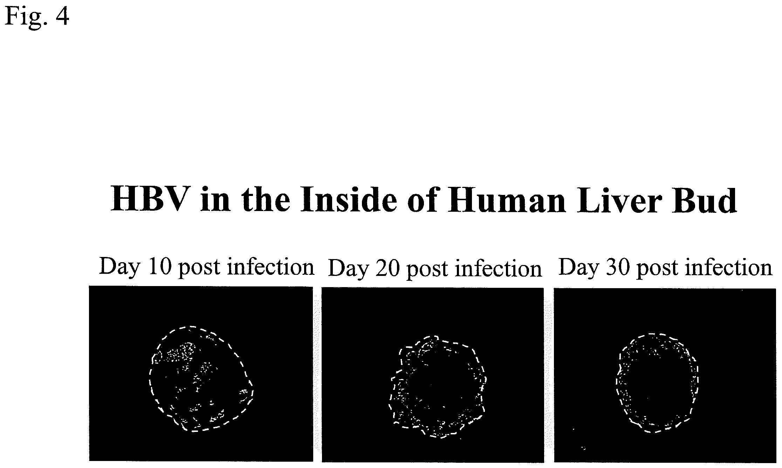

Fig. 4 shows immunostaining of the inside of human liver buds with HBc antibody at days 10, 20 and 30 post infection. HBV infection was noted continuously at days 10, 20 and 30 post infection.

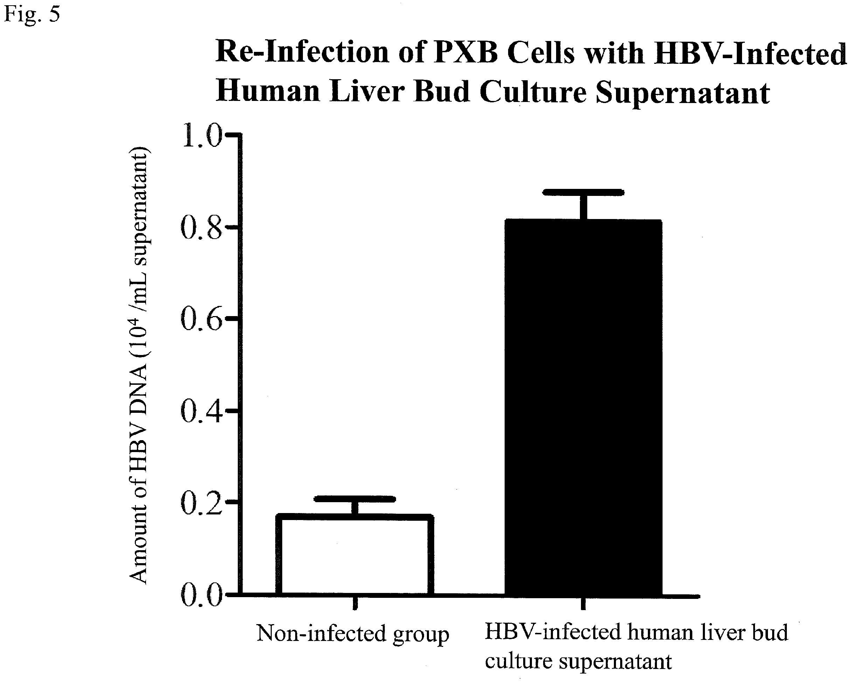

Fig. 5 is a graph comparing HBV DNA copy numbers in culture supernatants of primary human hepatocytes (PXB cells) between two cases where PXB cells were infected with the culture supernatant of human liver buds at day 20 post infection and with a non-infected group. It was revealed that HBV-infected human liver buds are capable of producing infectious HBV.

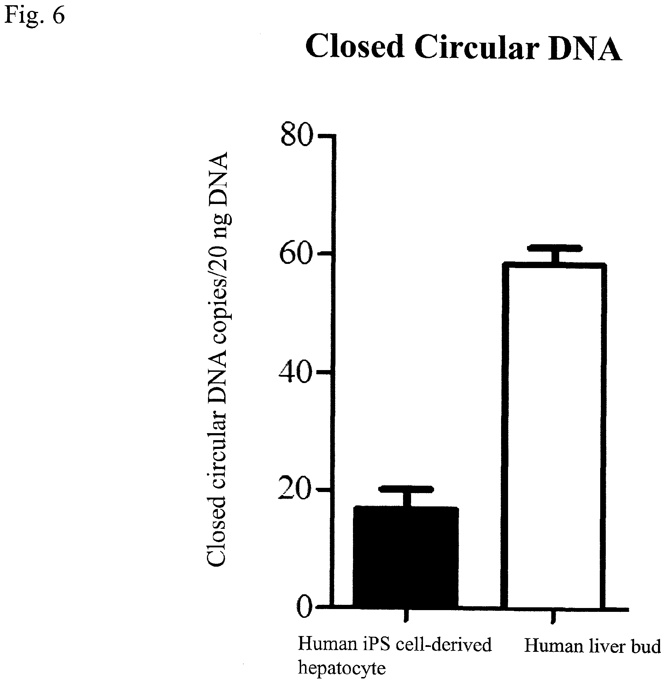

Fig. 6 is a graph comparing intracellular HBV covalently closed circular DNA copy numbers between two cases where human iPS cell-derived hepatocytes and human liver buds were infected with HBV. A markedly great number of HBV covalently closed circular DNA copies were noted in human liver buds, compared with human iPS cell-derived hepatocytes.

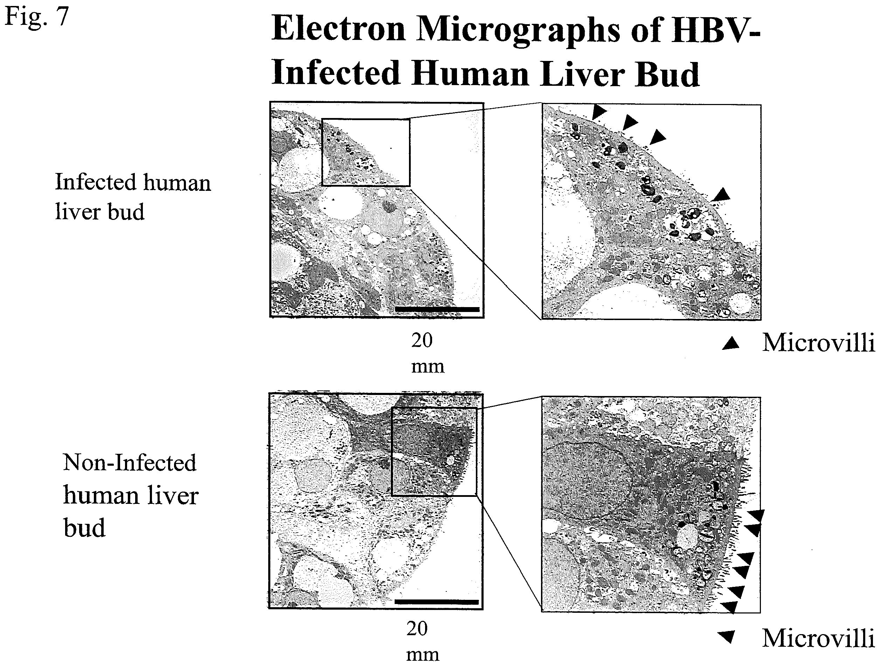

Fig. 7 is a set of electron micrographs comparing human liver bud at day 10 post infection with non-infected human liver bud. The HBV-infected human liver bud was observed to have fewer hepatocyte microvilli (indicated by arrow heads).

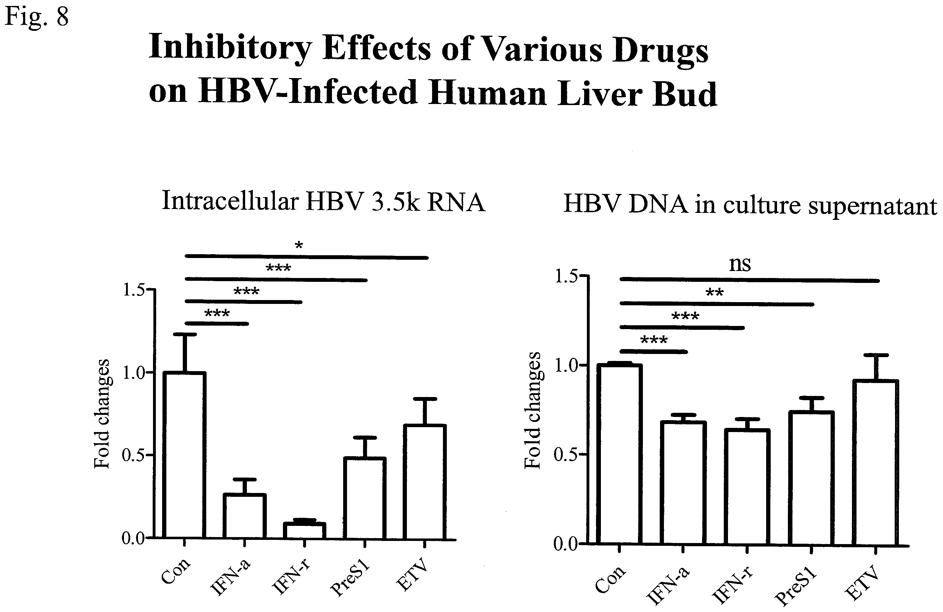

Fig. 8 shows that infection of human liver bud with HBV is inhibited by various drugs. Con: control group; IFN-α: interferon α 1000U/ml; IFN-γ: interferon γ 1000U/ml; PreS1: HBV surface antigen PreS1 peptide 100 nM; ETV: entecavir 1.8 µM. *p<0.05, **p<0.01, ***p<0.0001 vs Con.

### INDUSTRIAL APPLICABILITY

The present invention is applicable to *in vitro* screenings in drug discovery.

## Claims

1. A method for constructing a hepatitis virus infection model capable of recapitulating a viral life cycle, by infecting a liver bud with hepatitis virus, wherein said liver bud is formed by co-culturing liver cells derived from human iPSC with human vascular endothelial cells and human mesenchymal cells.

2. The method of claim 1, wherein the vascular endothelial cells are human umbilical vein endothelial cells.

3. The method of claim 1, wherein the mesenchymal cells are human bone marrow-derived mesenchymal cells.

4. The method of claim 1, wherein the infecting hepatitis virus is hepatitis B virus, hepatitis C virus or hepatitis E virus.

5. A hepatitis virus infection model capable of recapitulating a viral life cycle, comprising a hepatitis virus-infected liver bud, wherein the liver bud is formed by co-culturing liver cells derived from human iPSC with human vascular endothelial cells and human mesenchymal cells.

6. The virus infection model of claim 5, wherein the infecting hepatitis virus is hepatitis B virus, hepatitis C virus or hepatitis E virus.

7. A method of screening for substances with antiviral activity, comprising using the virus infection model of claim 5.

8. A method of screening for substances with antiviral activity, comprising using the virus infection model of claim 6, wherein the infecting virus is hepatitis B virus, hepatitis C virus or hepatitis E virus.

## Patentansprüche

1. Verfahren zum Aufbau eines Hepatitis-Virus-Infektionsmodells, das einen viralen Lebenszyklus durch Infektion einer Leberknospe mit Hepatitis-Virus rekapitulieren kann, wobei die Leberknospe durch Co-Kultivieren von Leberzellen, die von menschlichem iPSC stammen, mit menschlichen Gefäßendothelzellen und menschlichen mesenchymalen Zellen gebildet wird.

2. Verfahren nach Anspruch 1, wobei die Gefäßendothelzellen menschliche Endothelzellen der Nabelschnurvene sind.

3. Verfahren nach Anspruch 1, wobei die mesenchymalen Zellen aus menschlichem Knochenmark stammende mesenchymale Zellen sind.

4. Verfahren nach Anspruch 1, wobei das infizierende Hepatitis-Virus das Hepatitis-B-Virus, das Hepatitis-C-Virus oder das Hepatitis-E-Virus ist.

5. Hepatitis-Virus-Infektionsmodell, das einen viralen Lebenszyklus rekapitulieren kann und eine mit Hepatitis-Virus infizierte Leberknospe umfasst, wobei die Leberknospe durch Co-Kultivieren von Leberzellen, die von menschlichem iPSC stammen, mit menschlichen Gefäßendothelzellen und menschlichen mesenchymalen Zellen gebildet wird.

6. Virusinfektionsmodell nach Anspruch 5, wobei das infizierende Hepatitis-Virus das Hepatitis-B-Virus, das Hepatitis-C-Virus oder das Hepatitis-E-Virus ist.

7. Verfahren zum Screening auf Substanzen mit antiviraler Aktivität, das die Verwendung des Virusinfektionsmodells nach Anspruch 5 umfasst.

8. Verfahren zum Screening auf Substanzen mit antiviraler Aktivität, das die Verwendung des Virusinfektionsmodells nach Anspruch 6 umfasst, wobei das infizierende Virus das Hepatitis-B-Virus, das Hepatitis-C-Virus oder das Hepatitis-E-Virus ist.

## Revendications

1. Procédé de construction d'un modèle d'infection par le virus de l'hépatite capable de récapituler un cycle de vie viral, en infectant un bourgeon hépatique avec le virus de l'hépatite, dans lequel ledit bourgeon hépatique est formé en co-cultivant des cellules hépatiques dérivées d'iPSC humaine avec des cellules endothéliales vasculaires humaines et des cellules mésenchymateuses humaines.

2. Procédé selon la revendication 1, dans lequel les cellules endothéliales vasculaires sont des cellules endothéliales de veine ombilicale humaine.

3. Procédé selon la revendication 1, dans lequel les cellules mésenchymateuses sont des cellules mésenchymateuses dérivées de moelle osseuse humaine.

4. Procédé selon la revendication 1, dans lequel le virus infectant de l'hépatite est le virus de l'hépatite B, le virus de l'hépatite C ou le virus de l'hépatite E.

5. Modèle d'infection par le virus de l'hépatite capable de récapituler un cycle de vie viral, comprenant un bourgeon hépatique infecté par le virus de l'hépatite, dans lequel le bourgeon hépatique est formé par la co-culture de cellules hépatiques dérivées d'iPSC humaine avec des cellules endothéliales vasculaires humaines et des cellules mésenchymateuses humaines.

6. Modèle d'infection virale selon la revendication 5, dans lequel le virus infectant de l'hépatite est le virus de l'hépatite B, le virus de l'hépatite C ou le virus de l'hépatite E.

7. Procédé de criblage de substances à activité antivirale, comprenant l'utilisation du modèle d'infection virale selon la revendication 5.

8. Procédé de criblage de substances à activité antivirale, comprenant l'utilisation du modèle d'infection virale selon la revendication 6, dans lequel le virus infectant est le virus de l'hépatite B, le virus de l'hépatite C ou le virus de l'hépatite E.
